(19) Europäisches Patentamt
European Patent Office
Office européen des brevets

(11) EP 2 537 519 B1

(12) **EUROPEAN PATENT SPECIFICATION**

(45) Date of publication and mention of the grant of the patent:
**05.04.2017 Bulletin 2017/14**

(21) Application number: **11744531.2**

(22) Date of filing: **04.02.2011**

(51) Int Cl.:
*A61K 31/665* (2006.01) *A61P 25/04* (2006.01)
*A61K 31/683* (2006.01) *A61P 29/00* (2006.01)

(86) International application number:
**PCT/JP2011/052366**

(87) International publication number:
**WO 2011/102243 (25.08.2011 Gazette 2011/34)**

(54) **NEUROPATHIC PAIN ALLEVIATING AGENT**

MITTEL ZUR LINDERUNG NEUROPATHISCHER SCHMERZEN

AGENT D'ATTÉNUATION DE LA DOULEUR NEUROPATHIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.02.2010 JP 2010034766**

(43) Date of publication of application:
**26.12.2012 Bulletin 2012/52**

(73) Proprietors:
- **HBC Science Laboratory Co., Ltd**
  **Osaka-shi, Osaka 537-0021 (JP)**
- **Kinki University**
  **Higashiosaka-shi, Osaka 577-0818 (JP)**

(72) Inventors:
- **SHIBAYAMA Hiroharu**
  **Osaka-shi**
  **Osaka 537-0021 (JP)**
- **KAWABATA Atsufumi**
  **Higashiosaka-shi**
  **Osaka 577-0818 (JP)**

(74) Representative: **Grünecker Patent- und Rechtsanwälte**
**PartG mbB**
**Leopoldstraße 4**
**80802 München (DE)**

(56) References cited:
**EP-A1- 1 106 175** **WO-A1-2005/092905**
**US-A1- 2006 264 407**

- **OKUBO KAZUMASA ET AL: "Ascorbic acid inhibits hydrogen sulfide-induced hyperalgesia and paclitaxel-induced neuropathic pain", JOURNAL OF PHARMACOLOGICAL SCIENCES, JAPANESE PHARMACOLOGICAL SOCIETY, TOKYO, JP, vol. 109, no. Suppl. 1, 1 January 2009 (2009-01-01), page 222P, XP008163142, ISSN: 1347-8613**
- **CHEN JEN-YIN ET AL: "Plasma vitamin C is lower in postherpetic neuralgia patients and administration of vitamin C reduces spontaneous pain but not brush-evoked pain", CLINICAL JOURNAL OF PAIN, NEW YORK, NY, US, vol. 25, no. 7, 1 September 2009 (2009-09-01), pages 562-569, XP008163144, ISSN: 0749-8047, DOI: DOI:10.1097/AJP.0B013E318193CF32**
- **OKUBO KAZUMASA ET AL: "Effects of ascorbic acid and a novel amphiphilic ascorbic derivative on the hydrogen sulfide-induced hyperalgesia and paclitaxel-induced neuropathic", JOURNAL OF PHARMACOLOGICAL SCIENCES, JAPANESE PHARMACOLOGICAL SOCIETY, TOKYO, JP, vol. 112, no. Supllement 1, 16 March 2010 (2010-03-16), page 56p, XP008163140, ISSN: 1347-8613**
- **OKUBO KAZUMASA ET AL: "Application of disodium isostearyl 2-O-l-ascorbyl phosphate, a novel skin-permeable prodrug of ascorbic acid, as an ointment to treatment of neuropathic pain", JOURNAL OF PHARMACOLOGICAL SCIENCES, JAPANESE PHARMACOLOGICAL SOCIETY, TOKYO, JP, vol. 112, no. Supplement 1, 16 March 2010 (2010-03-16), page 130p, XP008163139, ISSN: 1347-8613**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 537 519 B1

• **OKUBO, K. ET AL.: 'Ascorbic acid inhibits hydrogen sulfide-induced hyperalgesia and paclitaxel-induced neuropathic pain' JOURNAL OF PHARMACOLOGICAL SCIENCES vol. 109, no. SUP.1, 2009, page 222P, XP008163142**

Remarks:

The file contains technical information submitted after the application was filed and not included in this specification

## Description

[Technical Field]

The present invention relates to a drug for inhibiting neuropathic pain which improves the symptom of neuropathic pain, or is used in treating a disease having the same symptom.

[Background Art]

Generally, treatment of the pain state in the medical field is very important, and a so-called pain therapy has been worldwidely demanded.

The urgent requirements with respect to specified treatment of the pain state or treatment of the specified pain state, which is suitable for a patient, are exemplified in a number of scientific works authored in recent years and over years, in the field of applied analgesic, or based on the basic study regarding sense of pain.

Pain is defined as "unpleasant sensory and emotional experience associated with actual or latent tissue damage" by International Association for the Study of Pain (IASP), or is described regarding such damage. In addition, pain is usually subjective, but the cause or syndrome thereof can be classified. Neuropathic pain as a subtype is defined as "pain initiated or caused by a primary lesion or functional failure of a nerve system" by IASP (IASP, Classification of chronic pain, 2nd Edition, IASP Press (2002), 210).

Neuropathic pain (neuropathy) is refractory pain generated by damage or a functional disorder of peripheral and central nerves and, in clinic, diabetic neuropathy, pathogenic pain such as postherpetic neuralgia or trigeminal neuralgia, and pain generated by the side effect due to drugs such as an anti-cancer agent are well-known.

For this neuropathic pain, the existing analgesics such as NSAIDs and Opioids are said to exert their effects with difficulty, and development of therapeutics which are clinically effective has become an urgent need. Currently, clinically, utilization of an antidepressant or an anti-epilepsy has been tried for neuropathic pain.

As shown in the following Table 1, in Japan, since an antidepressant and an anti-epilepsy are not recognized in insurance application for pain, except that Tegretol R (Carbamazepine) is recognized in application to trigeminal neuralgia, they are formulated as an analgesic assistant.

[Table 1]

| | |
|---|---|
| Antidepressant | Tricyclic antidepressant such as Tofranil (Imipramine) and Tryptanol (Amitriptyline)<br>Tetracyclic antidepressant such as Ludiomil (Maprotiline)<br>SSRI such as Paxil (Paroxetine)<br>SNRI such as Toledomin (Milnacipran) |
| anticonvulsant | Rivotril (Clonazepam), Tegretol (Carbamazepine, internal insurance application for trigeminal neuralgia), Gabapen (Gabapentin), Lyrica (Pregabalin, not approved in Japan) |

On the other hand, in USA and Europe, Gabapentin which is an anti-epilepsy is recognized in application to postherpetic neuralgia, and Pregabalin which is an analogous drug is approved as a therapeutic for neuropathic pain.

According to the above description, development of a therapeutic for neuropathic pain has been just started, but in Japan, development thereof is delayed under current circumstances. In addition, the drugs for which application to treatment of neuropathic pain has been tried currently are all oral drugs and, as a liniment, in Europe and USA, there is a use example of capsaicin cream (pepper ingredient), but in Japan, the cream is not commercially sold, and effectiveness thereof has not been sufficiently established.

In Kinki University, School of Pharmacy, Division of Pharmacology and Pathophysiology, involvement of hydrogen sulfide ($H_2S$) which is a gaseous information transmitting substance enzymatically produced in a living body, in a pain sense has been examined, and it has been found out that hyperalgesia is induced by administering NaHS which is a $H_2S$ donor to rats or mice intraplanterly or intraspinally, and it has been revealed that this effect is generated via activation of a T-type $Ca^{2+}$ channel which is present on a sensory nerve.

On the other hand, it has been certificated by a patch clamp method that, in a cultured neural cell, NaHS increases a membrane current via a T-type $Ca^{2+}$ channel. It has been made clear by a $Ca_v3.2$ gene knockdown method using an antisense method that hyperalgesia due to NaHS is caused via $Ca_v3.2$ among three subtypes of a T-type $Ca^{2+}$ channel, $Ca_v3.1$, 3.2 and 3.3 (Kawabata et al., Pain 2007; 132: 74-81, Maeda et al., Pain 2009; 142: 127.).

Further, it has been revealed that hydrogen sulfide/$Ca_v3.2$ T-type $Ca^{2+}$ channel system is involved in manifestation of neuropathic pain (Takahashi et al., J Pharmacol Sci 2009; 109 (Suppl 1): 190) in addition to pain accompanied with pancreatitis and intestinal pain (Matsunami et al., Gut 2009; 58: 751., Nishimura et al., Gut 2009; 58: 762.).

In addition, Nelson et al. (Nelson et al., J Neurosci 2007; 27: 12577-83) have reported that ascorbic acid specifically inhibits $Ca_v3.2$ among T-type $Ca^{2+}$ channels (Non-Patent Document 1) and okubo et al (Okubo et al, Journal of pharmaceutical sciences 2009; vol 109 suppl 1: 222P) have found that ascorbic acid inhibits hydrogen-induced hyperalgesia and paclitaxel-induced neuropathic pain. However, since ascorbic acid is very unstable to heat and oxidation, and is easily inactivated or degraded, the sufficient physiological action is not necessarily obtained, in some cases.

In order to stabilize L-ascorbic acid which is easily oxidized and is unstable as described above, a derivative in which an easily oxidized diol part is phosphate-esterified is known (Patent Document 1), and a derivative in which the part is glycosidated is also known (Patent Document 2).

In addition, an ascorbic derivative (see Chemical formula 1 described later) including an L-ascorbic acid-2-phosphoric acid ester in which a phosphoric acid ester part has a branched alkyl group, or a salt thereof, is known for its preferable permeability to a living body as a whitening cosmetic (Patent Document 3).

[Prior Art Documents]

[Patent Documents]

[Patent Document 1] JP-B No.52-18191
[Patent Document 2] JP-A No.03-139288
[Patent Document 3] WO 2005/092905

[Non Patent Document]

[Non-Patent Document 1] Nelson et al., Journal of Neuroscience 2007, vol.27, p.12577-12583

[Summary of the Invention]

[Problems to be Solved by the Invention]

However, whether an ascorbic derivative represented by the following Chemical formula 1 specifically inhibits $Ca_v3.2$ among T-type $Ca^{2+}$ channels equally with ascorbic acid has not been recognized, or the pharmacological action due to the derivative has not been recognized, and inhibition of neuropathic pain, that is, utilization as an ingredient for a medicament having the analgesic action has not been performed at all.

Then, an object of the present invention is to provide a novel ingredient having the action of inhibiting neuropathic pain, and utilize this ingredient effectively to obtain a novel drug for inhibiting neuropathic pain.

[Means to solve the problems]

The present inventors have paid an attention to the finding that a $Ca_v3.2$ T-type $Ca^{2+}$ channel present on a sensory nerve is also involved in manifestation of neuropathic pain, and have found out that a specified ascorbic acid derivative specifically and remarkably has the $Ca_v3.2$ T-type $Ca^{2+}$ channel inhibiting activity, resulting in completion of the present invention.

That is, in the present invention, the aforementioned problem was solved by a drug for inhibiting neuropathic pain containing, as an active ingredient, one or more kinds of ascorbic acid derivatives including an L-ascorbic acid-2-phosphoric acid ester which is represented by the following Chemical formula 1, and in which a phosphoric acid ester part has a branched alkyl group, or salt thereof.

[Chemical formula 1]

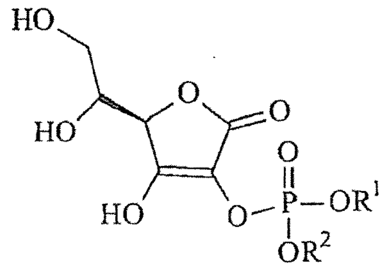

(wherein $R^1$ and $R^2$ represent hydrogen (H) or an alkyl group having a carbon number of 3 to 30 which is branched, provided that the case where $R^1$=$R^2$=hydrogen (H) is excluded)

Since the ascorbic acid derivatives have a phosphoric acid ester part at a predetermined position (2-position) of a molecular structure, and have a branched alkyl group at the part, the derivatives have a feature that they have suitable lipophilicity, and are easily taken up into a living body. Particularly, when $R^1$ or $R^2$ in Chemical formula 1 is an alkyl group having a carbon number of 4 to 30 which is branched at the 2-position, the above feature is remarkably exerted.

Representative examples of the ascorbic acid derivatives having the preferable action as described above include those in which the branched alkyl group is a 2-heptylundecyl group, a 2-octyldecyl group, a 2-octyldodecyl group, 2-hexyldecyl group, a 2-hexyldodecyl group, a 2-isoheptylisoundecyl group, a 16-methylheptadecyl group or a 2-(1,3,3-trimethyl-n-butyl)-5,7,7-trimethyl-n-octyl group.

Similarly, in order to obtain a salt of the ascorbic acid derivatives having the preferable action as described above, an inorganic salt such as a sodium salt, a potassium salt, a magnesium salt or a calcium salt of the L-ascorbic acid-2-phosphoric acid ester, or an organic salt including L-arginine, L-lysine, guanine, or basic organic amines is preferable.

[Effect of the Invention]

Since the present invention is a drug for inhibiting neuropathic pain containing, as an active ingredient, one or more kinds of ascorbic acid derivatives including an L-ascorbic acid-2-phosphiric acid ester, in which a phosphoric acid ester part has a branched alkyl group, or a salt thereof, the specific inhibitory effect for $Ca_v3.2$ among T-type $Ca^{2+}$ channels in a living body can be sufficiently manifested, and the drug has an advantage that it becomes a novel drug for inhibiting neuropathic pain having the action of inhibiting neuropathic pain.

[Brief Description of the Drawings]

Fig. 1 shows the pain inhibiting effect of Example 1, and a diagram (a) shows a relationship between a nociceptive threshold (%) and an elapsed time after application (min), and a diagram (b) shows an area under time threshold curve in the same diagram.

Fig. 2 shows the pain inhibiting effect of Comparative Example 1, and a diagram (a) shows a relationship between a nociceptive threshold (%) and an elapsed time after application (min), and a diagram (b) shows an area under time threshold curve in the same diagram.

Fig. 3 shows the pain inhibiting effect of Comparative Example 2, and a diagram (a) shows a relationship between a nociceptive threshold (%) and an elapsed time after application (min), and a diagram (b) shows an area under time threshold curve in the same diagram.

Fig. 4 shows the pain inhibiting effect of Comparative Example 3, and a diagram (a) shows a relationship between a nociceptive threshold (%) and an elapsed time after application (min), and a diagram (b) shows an area under time threshold curve in the same diagram.

Fig. 5 shows the pain inhibiting effects of Examples 2 and 3, and a diagram (a) shows a relationship between a nociceptive threshold (%) and an elapsed time after application (min), and a diagram (b) shows an area under time threshold curve in the same diagram.

Fig. 6 shows the pain inhibiting effects of Comparative Examples 4 and 5, and a diagram (a) shows a relationship between a nociceptive threshold (%) and an elapsed time after application (min), and a diagram (b) shows an area under time threshold curve in the same diagram.

[Mode for carrying out the invention]

An L-ascorbic acid-2-phosphoric acid ester shown by Chemical formula 1, that is, 2-(branched-type alkyl)-L-ascorbyl phosphate used in the present invention can be generally produced by the following method, and the details thereof are as described in Patent Document 3.

First, a branched alkanol and phosphorus oxychloride are reacted at -20 to 20°C using a non-polar solvent selected from toluene, chlorobenzene, dichlorobenzene, etc. in the presence of a base selected from trimethylamine, triethylamine, N,N-dimethylaniline, etc., to produce monoalkyl dichlorophosphate or dialkyl monochlorophosphate. Taking out may be isolation by distillation, or a solution of the non-polar solvent may proceed to a next step.

Further, 5,6-O-isopropylidene-L-ascorbic acid obtained by reacting L-ascorbic acid and acetone separately, and the monoalkyl dichlorophosphate or dialkyl monochlorophosphate obtained by the above step are reacted, and this reaction product is hydrolyzed with an acid, and purified by a conventional method, and thus, the L-ascorbic acid-2-phosphoric acid ester represented by Chemical formula 1 can be produced.

Specific compound examples of the L-ascorbic acid-2-phosphoric acid ester are as follows, and include 2-(2-methyldecyl)-L-ascorbyl phosphate, 2-(2-ethyldecyl)-L-ascorbyl phosphate, 2-(2-propyldecyl)-L-ascorbyl phosphate, 2-(2-butyldecyl)-L-ascorbyl phosphate, 2-(2-pentyldecyl)-L-ascorbyl phosphate, 2-(2-hexyldecyl)-L-ascorbyl phosphate, 2-(2-heptyldecyl)-L-ascorbyl phosphate, 2-(2-octyldecyl)-L-ascorbyl phosphate, 2-(2-nonyldecyl)-L-ascorbyl phosphate, 2-(2-methylundecyl)-L-ascorbyl phosphate, 2-(2-ethylundecyl)-L-ascorbyl phosphate, 2-(2-propylundecyl)-L-ascorbyl phosphate, 2-(2-butylundecyl)-L-ascorbyl phosphate, 2-(2-pentylundecyl)-L-ascorbyl phosphate, 2-(2-hexylundecyl)-L-ascorbyl phosphate, 2-(2-heptylundecyl)-L-ascorbyl phosphate, 2-(2-octylundecyl)-L-ascorbyl phosphate, 2-(2-nonylundecyl)-L-ascorbyl phosphate, 2-(2-decylundecyl)-L-ascorbyl phosphate, 2-(2-methyldodecyl)-L-ascorbyl phosphate, 2-(2-ethyldodecyl)-L-ascorbyl phosphate, 2-(2-propyldodecyl)-L-ascorbyl phosphate, 2-(2-butyldodecyl)-L-ascorbyl phosphate, 2-(2-pentyldodecyl)-L-ascorbyl phosphate, 2-(2-hexyldodecyl)-L-ascorbyl phosphate, 2-(2-heptyldodecyl)-L-ascorbyl phosphate, 2-(2-octyldodecyl)-L-ascorbyl phosphate, 2-(2-nonyldodecyl)-L-ascorbyl phosphate, 2-(2-decyldodecyl)-L-ascorbyl phosphate, 2-(2-undecyldodecyl)-L-ascorbyl phosphate, 2-(2-isoheptylisoundecyl)-L-ascorbyl phosphate, 2-(16-methylheptadecyl)-L-ascorbyl phosphate, 2-bis(2-methyldecyl)-L-ascorbyl phosphate, 2-bis(2-ethyldecyl)-L-ascorbyl phosphate, 2-bis(2-propyldecyl)-L-ascorbyl phosphate, 2-bis(2-butyldecyl)-L-ascorbyl phosphate, 2-bis(2-pentyldecyl)-L-ascorbyl phosphate, 2-bis(2-hexyldecyl)-L-ascorbyl phosphate, 2-bis(2-heptyldecyl)-L-ascorbyl phosphate, 2-bis(2-octyldecyl)-L-ascorbyl phosphate, 2-bis(2-nonyldecyl)-L-ascorbyl phosphate, 2-bis(2-methylundecyl)-L-ascorbyl phosphate, 2-bis(2-ethylundecyl)-L-ascorbyl phosphate, 2-bis(2-propylundecyl)-L-ascorbyl phosphate, 2-bis(2-butylundecyl)-L-ascorbyl phosphate, 2-bis(2-pentylundecyl)-L-ascorbyl phosphate, 2-bis(2-hexylundecyl)-L-ascorbyl phosphate, 2-bis(2-heptylundecyl)-L-ascorbyl phosphate, 2-bis(2-octylundecyl)-L-ascorbyl phosphate, 2-bis(2-nonylundecyl)-L-ascorbyl phosphate, 2-bis(2-decylundecyl)-L-ascorbyl phosphate, 2-bis(2-methyldodecyl)-L-ascorbyl phosphate, 2-bis(2-ethyldodecyl)-L-ascorbyl phosphate, 2-bis(2-propyldodecyl)-L-ascorbyl phosphate, 2-bis(2-butyldodecyl)-L-ascorbyl phosphate, 2-bis(2-pentyldodecyl)-L-ascorbyl phosphate, 2-bis(2-hexyldodecyl)-L-ascorbyl phosphate, 2-bis(2-heptyldodecyl)-L-ascorbyl phosphate, 2-bis(2-octyldodecyl)-L-ascorbyl phosphate, 2-bis(2-nonyldodecyl)-L-ascorbyl phosphate, 2-bis(2-decyldodecyl)-L-ascorbyl phosphate, 2-bis(2-undecyldodecyl)-L-ascorbyl phosphate, 2-bis(2-isoheptylisoundecyl)-L-ascorbyl phosphate, 2-bis(16-methylheptadecyl)-L-ascorbyl phosphate, 2-bis[2-(1,3,3-trimethyl-n-butyl)-5,7,7-trimethyl-n-octyl]-L-ascorbyl phosphate.

The compound has a branched alkyl group at the 2-position, and may contain a branched alkyl group at a position other than the above position, for example, 2-[2-(1,3,3-trimethyl-n-butyl)-5,7,7-trimethyl-n-octyl]-L-ascorbyl phosphate.

The ascorbic acid derivatives of the present invention all have the action of inhibiting neuropathic pain as described later, and a salt of the ascorbic acid derivative can be also used and, for example, alkali metal salts such as a sodium salt and a potassium salt, alkaline earth metal salts such as a calcium salt and a magnesium salt, basic amino acids such as arginine, and organic amines such as triethanolamine can be used.

The ascorbic acid derivatives used in the present invention are contained, as an active ingredient, in the drug for inhibiting neuropathic pain derived from specific inhibition of a $Ca_v3.2$ T-type $Ca^{2+}$ channel in a living body. Examples of such preparations include a skin external agent and a poultice.

An effective dose of ascorbic acid derivatives is different depending on a relative efficacy of a selected compound, severity of a disorder to be treated, and a weight of an affected individual and, daringly, as examples of the dose at which the effect is manifested, 10 nmol/paw or more is preferable. In addition, 20 to 60 nmol/paw adopted in examples described later can be exemplified as an effective dose example and, in addition, 10 to 2000 nmol/paw can be adopted as a preferable effective dose example.

In addition, when converted from the above numerical values, as an effective (application) dose of the ascorbic acid derivatives of the present invention, an external agent containing around 0.1 to 20% by weight as an effective concentration for an adult (about 60 kg) can be used by applying at appropriate times depending on the symptom, and an administration time of 1 to 10 times, usually around 5 to 6 times (several times) a day is preferable.

In addition, the drug for inhibiting neuropathic pain of the present invention has high safety to the same degree as that of a general medicament using ascorbic acid, and can be adopted in an arbitrary preparation form as described later and, at the same time, at least one kind of an auxiliary material or additive or both thereof can be used together with an active ingredient, similar to general medicament compositions.

That is, a dosage form of the external composition of the present invention may be altered depending on a demand or object of consumers, and a form of an external agent such as cream-like, emulsion-like, liquid, gel-like, ointment-like, pack-like, stick-like and powder-like forms can be selectively adopted.

In addition to the active ingredient, various components used in usual quasi drugs, medicaments etc., for example, oily components, emulsifiers, humectants, thickeners, drug efficacy components, antiseptics, pigments, powders, pH adjusting agents, ultraviolet adsorbing agents, antioxidants, perfumes etc. can be appropriately incorporated into the external composition of the present invention.

As the auxiliary material or additive, one or more kinds selected from carries, excipients, supporting materials, lubricants, fillers, solvents, diluents, coloring agents, taste adjusting agents such as sugars, antioxidants, and binders can be used.

In addition, the drug for inhibiting neuropathic pain of the present invention is directed to skin external use and, particularly, it is preferable that the drug takes a form of cream preparations or a liquid form.

The drug for inhibiting neuropathic pain used in such a preparation form permeates into the skin and is rapidly degraded into L-ascorbic acid and an acyl phosphate compound with phosphatase that is widely distributed in a living body, and has the excellent $Ca_v3.2$ T-type $Ca^{2+}$ channel inhibiting activity effect inherent to L-ascorbic acid.

[Examples]

[Examples 1 to 3 and Comparative Examples 1 to 5]

As an agent to be used as an active ingredient, amphoteric ascorbic acid (2-[2-(1,3,3-trimethyl-n-butyl)-5,7,7-trimethyl-n-octyl]-L-ascorbyl phosphate)(manufactured by HBC Science Laboratory Co., Ltd.: disodium isostearyl 2-0-1-ascorbyl phosphate)(hereinafter, abbreviated as DI-VCP), sodium hydrogen sulfide (manufactured by Kishida Chemical Co., Ltd.: NaHS) or ascorbic acid (Sigma Ltd. St Louis, MO, USA) was used to prepare the following preparations.

That is, an ointment with DI-VCP incorporated therein or an ointment with ascorbic acid incorporated therein was made by the following procedure.

First, 100 μL of a 0.8 or 2.4 mM DI-VCP aqueous solution was added per 1 g of 5% (w/w) Cetanol (manufactured by Kishida Chemical Co., Ltd.)-containing Solbase (manufactured by Meiji Yakuhin Co., Ltd.) to obtain an ointment. Similarly, an ointment preparation using a 2.4 mM ascorbic acid aqueous solution was also made.

The resulting ointment with DI-VCP incorporated therein (hereinafter, referred to as DI-VIP ointment) at a dose of 250 mg/paw (20 or 60 nmol/paw) or the ointment with ascorbic acid incorporated therein (hereinafter, referred to as ascorbic acid ointment) at a dose of 250 mg/paw (60 nmol/paw) was applied on a unilateral hind leg of a rat adopted as an experimental animal, respectively, the following experiment for measuring a mechanical nociceptive threshold, and a paclitaxel-induced neuropathic pain model experiment were performed, and the results were shown in Figs. 1 to 6.

As a rat for the experimental animal, a male Wistar rat (Japan SLC Inc, Hamamatsu) was used, and the rat ingested ad libitum a solid feed (manufactured by MF Oriental Yeast Co., Ltd.) and tap water, and was reared in a room retaining light / dark cycle at about 24 °C for 12 hours.

[Experiment for measuring mechanical nociceptive threshold]

Measurement of a mechanical nociceptive threshold was performed by the Randall-Selitto method. Using an apparatus for measuring a pressure stimulation analgesic effect (MK-300, Muromachi Kikai Co., Ltd., Tokyo), mechanical pressure stimulation of 30 g/s was given to a right hind leg of the rat, and a mechanical nociceptive threshold was measured employing a flounder reaction or bawl as an index. In addition, in order to prevent damage of a hind leg, a limit of pressure stimulation to be applied was set to be 500 g. The obtained result was shown by a value calculated by the following mathematical equation, allowing a mechanical nociceptive threshold before administration of a drug or before initiation of paclitaxel administration to be 100%.

Mechanical nociceptive threshold AIC (% baseline)={obtained threshold/(threshold before NaHS administration or before initiation of paclitaxel administration)}×100

[Making of paclitaxel-induced neuropathic pain model]

Making of a paclitaxel (hereinafter, abbreviated as PTX)-induced neuropathic pain model was performed according to the method of Polomano et al. (Polomano et al., Pain 2001; 94: 293-304). Before paclitaxel administration, a baseline threshold was measured, thereafter, 6 mg/mL of a paclitaxel injection solution (manufactured by Bristol-Myers Squibb Company) was diluted three times with a physiological saline, and a dose of 2 mg/kg was intraperitoneally administered to the rat at a total of four times, on day 0 which was the first administration and, subsequently, on days 2, 4 and 6 (total 8 mg/kg). Also before each administration, and on days 10 and 14, a pain sense threshold was measured, elapse was observed, reduction in the pain sense threshold was confirmed and, thereafter, this was used in an experiment thereafter. A solvent for a paclitaxel injection solution (Cremophor EL (polyethoxylated castor oil) / ethanol; 50 / 50%) was diluted three times, and this was administered to a control group according to the similar schedule.

[Administration method]

In a NaHS-induced neuropathic pain experiment, the DI-VCP ointment or the ascorbic acid ointment was applied at a dose of 60 nmol/paw and, after 90 minutes, NaHS was subcutaneously administered to a rat planta at a dose of 1 nmol/paw (10 μM solution, 100 μL/paw).

In a paclitaxel-induced neuropathic pain model rat experiment, the DI-VCP ointment or the ascorbic acid ointment was applied on a unilateral hind leg of the rat at a dose of 20 or 60 nmol/paw.

The results shown in Figs. 1 to 6 are summarized as follows:

[Regarding Example 1 and Comparative Example 1]

Figs. 1 and 2 show the effect of the ascorbic acid ointment and the DI-VCP ointment applications for hyperalgesia induced by NaHS plantar subcutaneous administration. NaHS at a dose of 1 nmol/paw and a physiological saline were subcutaneously administered to a planta at an administration volume of 100 μL/paw. The disodium isostearyl 2-0-1-ascorbyl phosphate (DI-VCP)-containing ointment or the ascorbic acid-containing ointment was applied on a surface of the rat right hind leg 90 minutes before NaHS administration at a dose of 60 nmol/paw (application amount 250 mg/paw). Each broken line in the figure indicates average ± standard error of 6 to 8 examples, and * and ** indicate a significant difference for a physiological saline-intraplantar administration group after application of the physiological saline-containing ointment ($P<0.05$, $P<0.01$, Tukey method). ## indicates a significant difference for a NaHS administration group after application of the physiological saline-containing ointment ($P<0.01$, Tukey method).

When the DI-VCP ointment was applied on a rat unilateral hind leg at a dose of 60 nmol/paw, hyperalgesia due to NaHS plantar subcutaneous administration was arrested (Fig. 1). On the other hand, when the ascorbic acid ointment was applied on a rat unilateral hind leg at a dose of 60 nmol/paw, hyperalgesia was not influenced by NaHS plantar subcutaneous administration (Fig. 2).

[Regarding Comparative Example 2 and Comparative Example 3]

Figs. 3 and 4 show the influence of application of the ascorbic acid ointment on paclitaxel-induced neuropathic pain. Paclitaxel was intraperitoneally administered at a dose of 2 mg/kg on day 0 which was the first administration and, thereafter, on days 2, 4 and 6. For the rat 14 days after paclitaxel administration, the ascorbic acid-containing ointment was applied on a surface of the rat right hind leg at a dose of 60 nmol/paw (application amount 250 mg/paw). Each broken line in the figure indicates average ± standard error of 5 to 7 examples, and * and ** indicate a significant difference for the physiological saline-containing ointment application group in a control ($P<0.05$, $P<0.01$, Tukey method).

When the ascorbic acid ointment was applied on a unilateral hind leg of the paclitaxel-treated rat at a dose of 60 nmol/paw, no influence on a pain sense threshold was recognized on both of the treatment side (Fig. 3) and the opposite side (Fig. 4).

[Regarding Examples 2 and 3 and Comparative Examples 4 and 5]

Figs. 5 and 6 show the effect of application of the DI-VCP ointment on paclitaxel-induced neuropathic pain. Paclitaxel was intraperitoneally administered at a dose of 2 mg/kg on day 0 which was the first administration and, thereafter, on days 2, 4 and 6. For the rat 14 days after paclitaxel administration, the ascorbic acid-containing ointment was applied on a surface of the rat right hind leg at a dose of 60 nmol/paw (application amount 250 mg/paw). Each broken line in the figure indicates average ± standard error of 5 to 7 examples, * and ** indicate a significant difference for the physiological saline-containing ointment application group in a control, and # and ## indicate a significant difference of the paclitaxel administration group relative to the physiological saline application group ($P<0.05$, $P<0.01$, Tukey

method).

When the DI-VCP ointment was applied on a rat unilateral hind leg at a dose of 20 nmol/paw (Example 2) and 60 nmol/paw (Example 3), increase in a pain sense threshold was recognized dose-dependently on a treatment side and, particularly, the significant analgesic effect was recognized from 80 minutes after application at 60 nmol/paw. This analgesic effect lasted over a long time of 4 hours or longer, and disappeared after 24 hours (Fig. 5). On the other hand, no influence on a pain sense threshold was seen on the application opposite side (Fig. 6).

Comprehensively from the foregoing results, conventional ascorbic acid which selectively inhibits a $Ca_v3.2$ T-type $Ca^{2+}$ channel was invalid even when locally applied as an ointment, while the present invention inhibited hyperalgesia induced by plantar subcutaneous administration of NaHS which activates $Ca_v3.2$ and, further, inhibited neuropathic pain induced by an anti-cancer agent (paclitaxel) for a long time.

Since it is reported that a T-type $Ca^{2+}$ channel is involved in not only neuropathic pain associated with cancer chemotherapy, but also the disease states of neuropathic pain and diabetic neuropathy made by a surgical procedure, it is thought that a DI-VCP local liniment (ointment etc,) can be clinically applied as a new therapeutic for neuropathic pain developed by various causes.

Therefore, it was confirmed that the drug for inhibiting neuropathic pain containing an ascorbic acid derivative of the present invention permeates into the skin and is rapidly degraded into L-ascorbic acid and an acyl phosphate compound with phosphatase that is widely distributed in a living body, and has the excellent $Ca_v3.2$ T-type $Ca^{2+}$ channel inhibitory activity effect inherent to L-ascorbic acid.

Representative formulation examples of the drug for inhibiting neuropathic pain which are example, and in which a predetermined ascorbic acid derivative used in the present invention is an active ingredient will be shown below. A numerical value at each line right end is an incorporation ratio (% by weight).

[Formulation Example 1] (External ointment)

| | |
|---|---|
| 2-[2-(1,3,3-Trimethyl-n-butyl)-5,7,7-trimethyl-n-octyl]-L-ascorbyl phosphate (DI-VCP) | 2.0 |
| Vaseline | 25.0 |
| Stearyl alcohol | 20.0 |
| Propylene glycol | 12.0 |
| Polyoxyethylene hardened castor oil | 4.0 |
| Glyceryl monostearate | 1.0 |
| Methyl paraoxybenzoate | 0.1 |
| Propyl paraoxybenzoate | q.s. |
| Purified water | Balance |

[Formulation Example 2] (Gel-like agent)

| | |
|---|---|
| 2-Bis(2-octyldodecyl)-L-ascorbyl phosphate | 5.0 |
| Glycerin | 15.0 |
| Ethanol | 5.0 |
| Carboxyvinyl polymer | 1.5 |
| Sodium hydroxide | 0.2 |
| Antiseptic | q.s. |
| Purified water | Balance |

[Formulation Example 3] (Liquid agent)

| | |
|---|---|
| 2-(2-Hexyldecyl)-L-ascorbyl phosphate | 5.0 |
| Glycerin | 10.0 |
| Propylene glycol | 5.0 |

(continued)

| | |
|---|---|
| Antiseptic | q.s. |
| Purified water | Balance |

**Claims**

1. A skin external agent for use in inhibiting neuropathic pain comprising, as an active ingredient, 0.1 to 20% by weight of one or more kinds of ascorbic acid derivatives represented by the following Chemical formula (1), or a salt thereof,

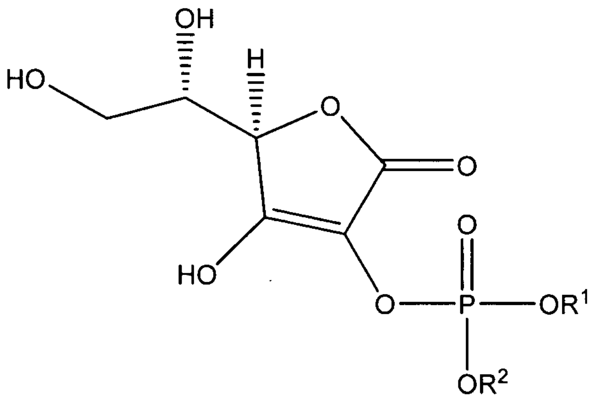

(1)

wherein $R^1$ and $R^2$ represent hydrogen or an alkyl group having a carbon number of 3 to 30 which is branched, provided that the case where $R^1 = R^2$ = hydrogen is excluded.

2. The skin external agent for use according to claim 1, wherein $R^1$ or $R^2$ in Chemical formula (1) is an alkyl group having a carbon number of 4 to 30 which is branched at the 2-position.

3. The skin external agent for use according to claim 1, wherein the branched alkyl group is a 2-heptylundecyl group, a 2-octyldecyl group, a 2-octyldodecyl group, a 2-hexyldecyl group, a 2-hexyldodecyl group, a 2-isoheptylisoundecyl group, a 16-methylheptadecyl group or a 2-(1,3,3-trimethyl-n-butyl)-5,7,7-trimethyl-n-octyl group.

4. The skin external agent for use according to any one of claims 1 to 3, wherein the salt is an inorganic salt including a sodium salt, a potassium salt, a magnesium salt or a calcium salt of the L-ascorbic acid-2-phosphoric acid ester.

5. The skin external agent for use according to any one of claims 1 to 3, wherein the salt is an organic salt including L-arginine, L-lysine, guanine or basic organic amines.

**Patentansprüche**

1. Äußerliches Hautmittel zur Verwendung bei der Verhinderung neuropathischer Schmerzen, umfassend als Wirkstoff 0,1 bis 20 Gew.-% einer oder mehrerer Arten von Ascorbinsäurederivaten, dargestellt durch die folgende chemische Formel (1), oder ein Salz davon,

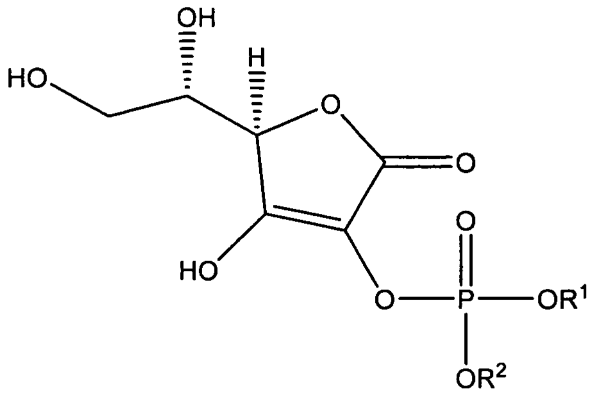

(1)

wobei $R^1$ und $R^2$ Wasserstoff oder eine Alkylgruppe mit einer Kohlenstoffzahl von 3 bis 30 darstellen, die verzweigt ist, mit der Maßgabe, dass $R^1 = R^2$ = Wasserstoff ausgeschlossen ist.

2. Äußerliches Hautmittel zur Verwendung gemäß Anspruch 1, wobei $R^1$ oder $R^2$ in der chemischen Formel (1) eine Alkylgruppe mit einer Kohlenstoffzahl von 4 bis 30 ist, die an Position 2 verzweigt ist.

3. Äußerliches Hautmittel zur Verwendung gemäß Anspruch 1, wobei die verzweigte Alkylgruppe eine 2-Heptylundecylgruppe, eine 2-Octyldecylgruppe, eine 2-Octyldodecylgruppe, eine 2-Hexyldecylgruppe, eine 2-Hexyldodecylgruppe, eine 2-Isoheptylisoundecylgruppe, eine 16-Methylheptadecylgruppe oder eine 2-(1,3,3-Trimethyl-n-butyl)-5,7,7-trimethyl-n-octylgruppe ist.

4. Äußerliches Hautmittel zur Verwendung gemäß irgendeinem der Ansprüche 1 bis 3, wobei das Salz ein anorganisches Salz, einschließlich eines Natriumsalzes, eines Kaliumsalzes, eines Magnesiumsalzes oder eines Calciumsalzes von L-Ascorbinsäure-2-phosphorsäureester ist.

5. Äußerliches Hautmittel zur Verwendung gemäß irgendeinem der Ansprüche 1 bis 3, wobei das Salz ein organisches Salz, einschließlich L-Arginin, L-Lysin, Guanin oder basischen organischen Aminen ist.

**Revendications**

1. Agent cutané externe pour l'utilisation dans l'inhibition de la douleur neuropathique comprenant, comme ingrédient actif, de 0, 1 à 20 % en poids d'un ou plusieurs types de dérivés d'acide ascorbique représenté par la formule chimique suivante (1), ou un sel de celui-ci,

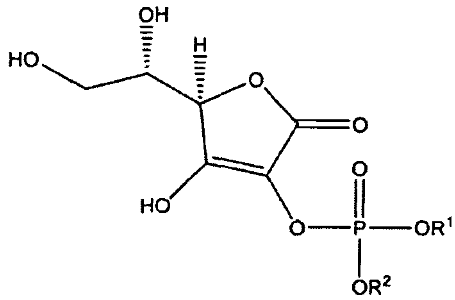

(1)

dans laquelle $R^1$ et $R^2$ représentent un atome d'hydrogène ou un groupe alkyle ayant un nombre d'atomes de carbone de 3 à 30 qui est ramifié, à condition que le cas où $R^1 = R^2$ = hydrogène soit exclu.

2. Agent cutané externe pour l'utilisation selon la revendication 1, dans lequel $R^1$ ou $R^2$ dans la formule chimique (1) est un groupe alkyle ayant un nombre d'atomes de carbone de 4 à 30 qui est ramifié à la position 2.

3. Agent cutané externe pour l'utilisation selon la revendication 1, dans lequel le groupe alkyle ramifié est un groupe 2-heptylundécyle, un groupe 2-octyldécyle, un groupe 2-octyldodécyle, un groupe 2-hexyldécyle, un groupe 2-hexyldodécyle, un groupe 2-isoheptylisoundécyle, un groupe 16-méthylheptadécyle ou un groupe 2-(1,3,3-triméthyl-*n*-butyl)-5,7,7-triméthyl-*n*-octyle.

4. Agent cutané externe pour l'utilisation selon l'une quelconque des revendications 1 à 3, dans lequel le sel est un sel inorganique incluant un sel de sodium, un sel de potassium, un sel de magnésium ou un sel de calcium de l'ester d'acide L-ascorbique-acide 2-phosphorique.

5. Agent cutané externe pour l'utilisation selon l'une quelconque des revendications 1 à 3, dans lequel le sel est un sel organique incluant la L-arginine, la L-lysine, la guanine ou les amines organiques basiques.

Fig.1

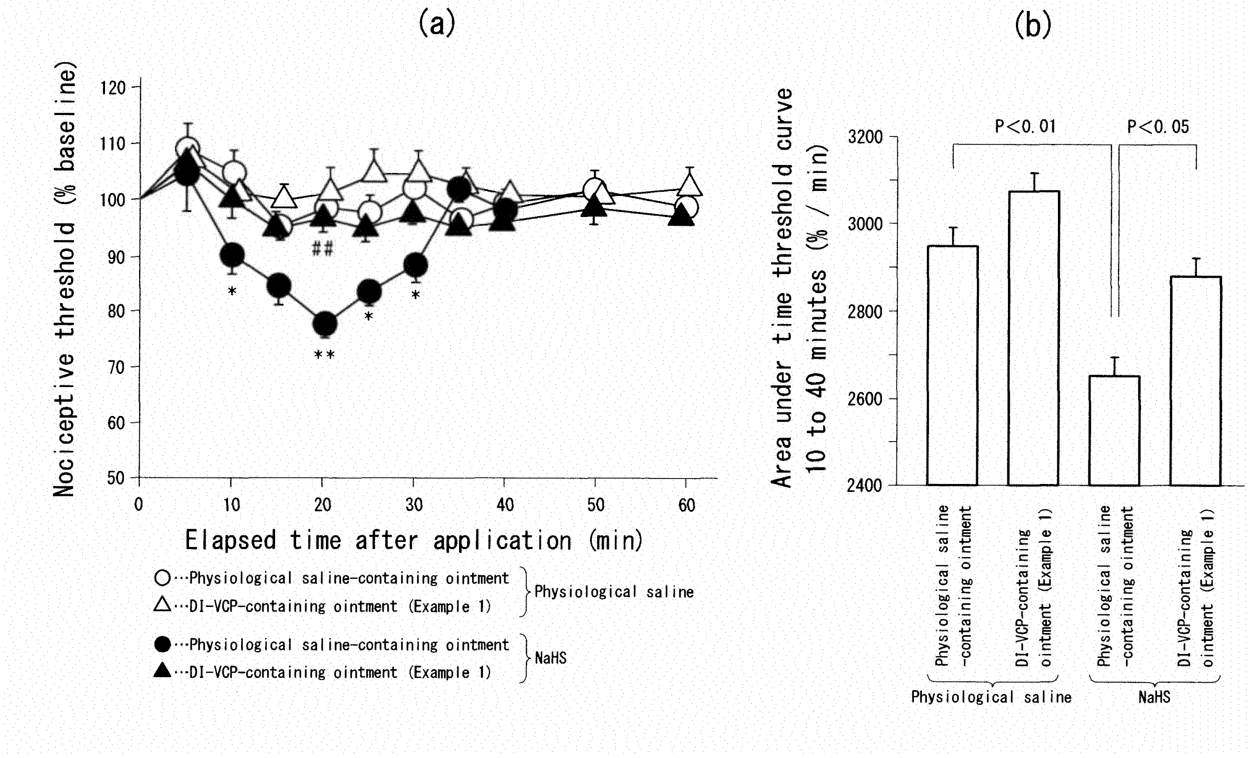
(a)
(b)
Nociceptive threshold (% baseline)
Elapsed time after application (min)
○···Physiological saline-containing ointment
△···DI-VCP-containing ointment (Example 1)
Physiological saline
●···Physiological saline-containing ointment
▲···DI-VCP-containing ointment (Example 1)
NaHS
Area under time threshold curve 10 to 40 minutes (% / min)
P<0.01
P<0.05
Physiological saline -containing ointment
DI-VCP-containing ointment (Example 1)
Physiological saline -containing ointment
DI-VCP-containing ointment (Example 1)
Physiological saline
NaHS

Fig.2

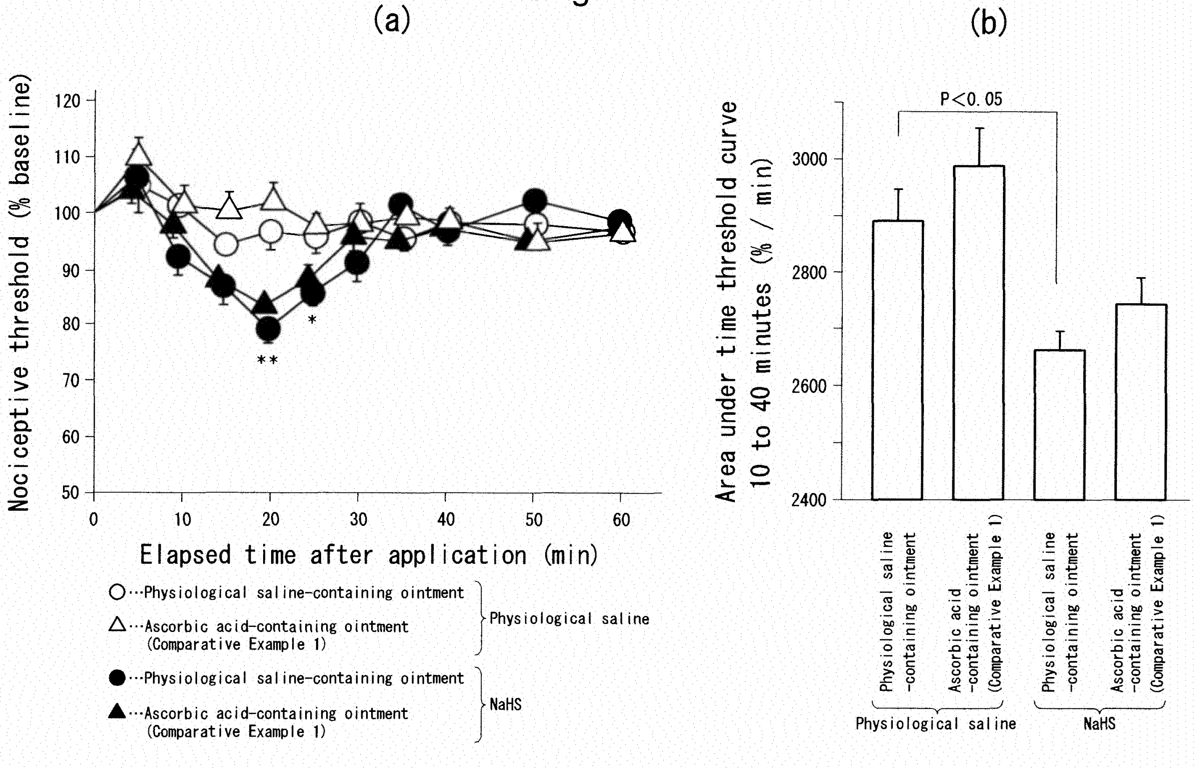
(a)
(b)
Nociceptive threshold (% baseline)
120
110
100
90
80
70
60
50
0
10
20
30
40
50
60
Elapsed time after application (min)
**
*
○···Physiological saline-containing ointment
△···Ascorbic acid-containing ointment (Comparative Example 1)
Physiological saline
●···Physiological saline-containing ointment
▲···Ascorbic acid-containing ointment (Comparative Example 1)
NaHS
Area under time threshold curve 10 to 40 minutes (% / min)
3000
2800
2600
2400
P<0.05
Physiological saline -containing ointment
Ascorbic acid -containing ointment (Comparative Example 1)
Physiological saline -containing ointment
Ascorbic acid -containing ointment (Comparative Example 1)
Physiological saline
NaHS

Fig.3

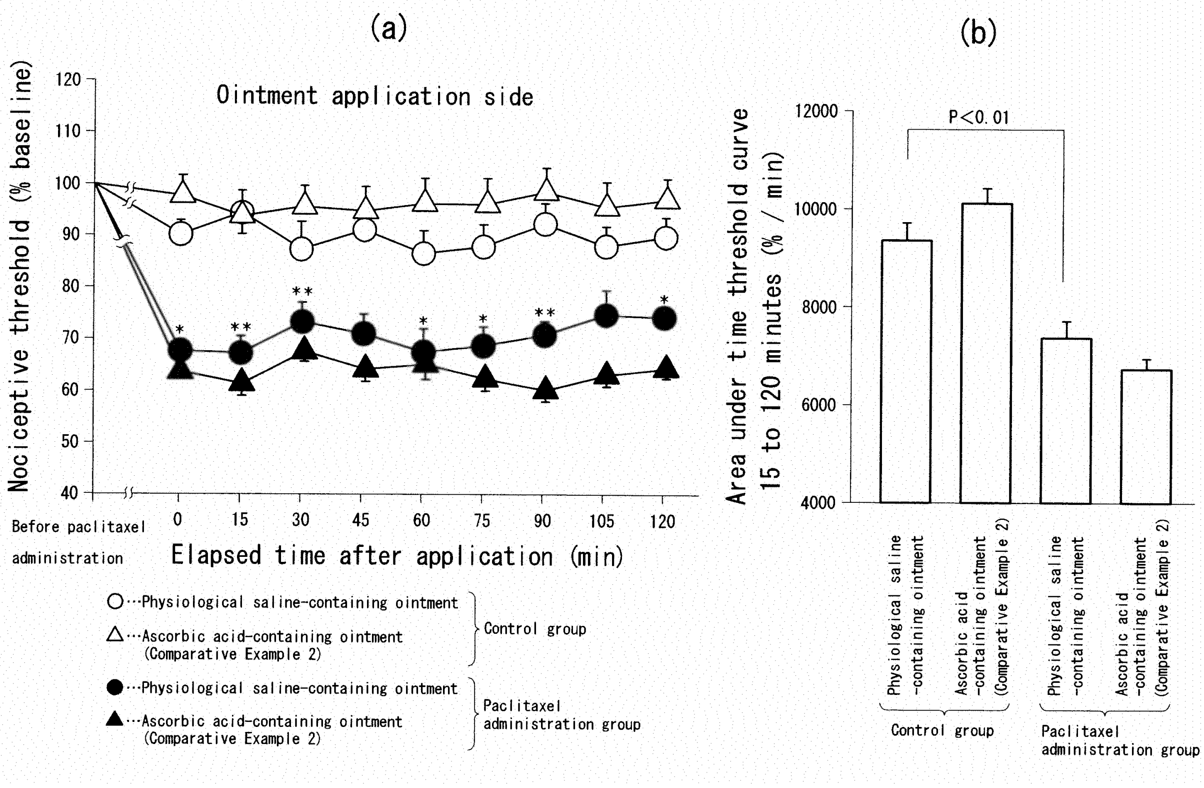
(a)
Ointment application side
Nociceptive threshold (% baseline)
120
110
100
90
80
70
60
50
40
Before paclitaxel administration
0
15
30
45
60
75
90
105
120
Elapsed time after application (min)
○···Physiological saline-containing ointment
△···Ascorbic acid-containing ointment (Comparative Example 2)
Control group
●···Physiological saline-containing ointment
▲···Ascorbic acid-containing ointment (Comparative Example 2)
Paclitaxel administration group
(b)
Area under time threshold curve 15 to 120 minutes (% / min)
12000
10000
8000
6000
4000
P<0.01
Physiological saline -containing ointment
Ascorbic acid -containing ointment (Comparative Example 2)
Physiological saline -containing ointment
Ascorbic acid -containing ointment (Comparative Example 2)
Control group
Paclitaxel administration group

Fig.4

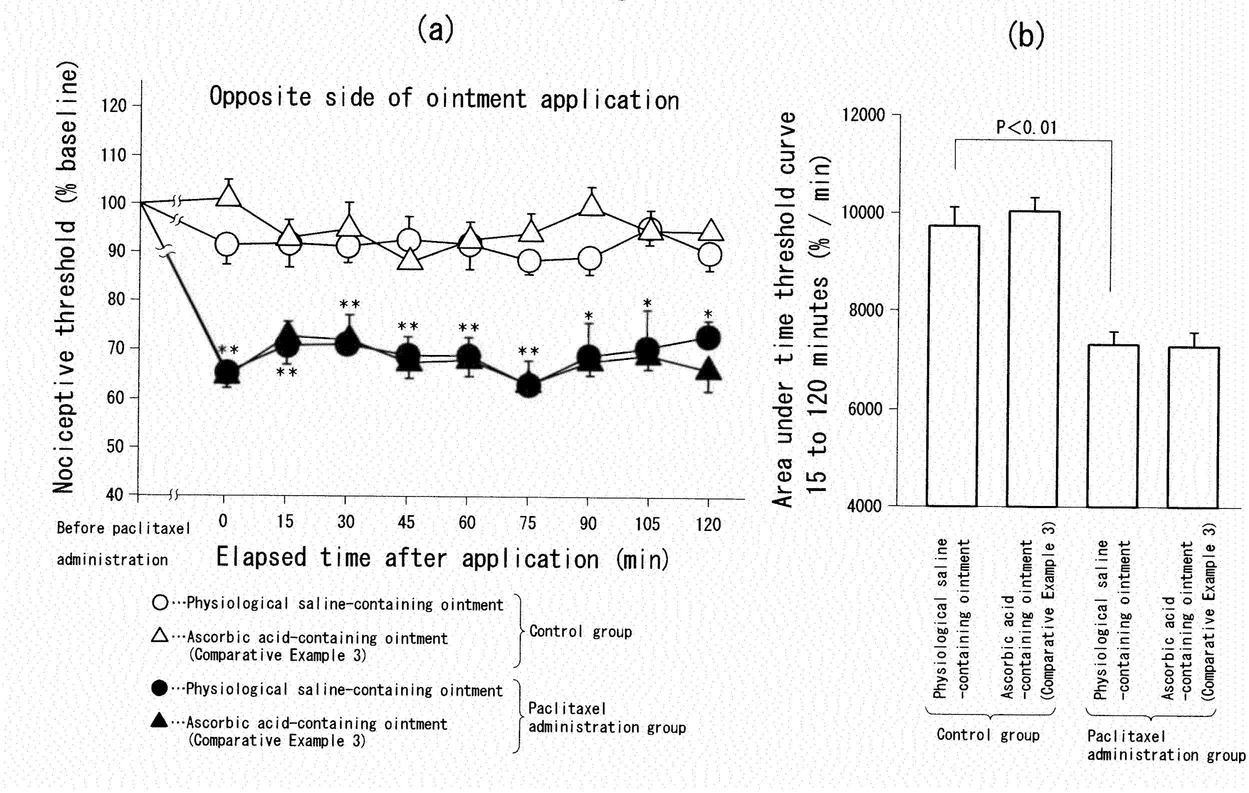
(a)
Opposite side of ointment application
Nociceptive threshold (% baseline)
120
110
100
90
80
70
60
50
40
Before paclitaxel administration
0
15
30
45
60
75
90
105
120
Elapsed time after application (min)
○···Physiological saline-containing ointment
△···Ascorbic acid-containing ointment (Comparative Example 3)
Control group
●···Physiological saline-containing ointment
▲···Ascorbic acid-containing ointment (Comparative Example 3)
Paclitaxel administration group
(b)
Area under time threshold curve 15 to 120 minutes (% / min)
12000
10000
8000
6000
4000
P<0.01
Physiological saline -containing ointment
Ascorbic acid -containing ointment (Comparative Example 3)
Physiological saline -containing ointment
Ascorbic acid -containing ointment (Comparative Example 3)
Control group
Paclitaxel administration group

Fig.5

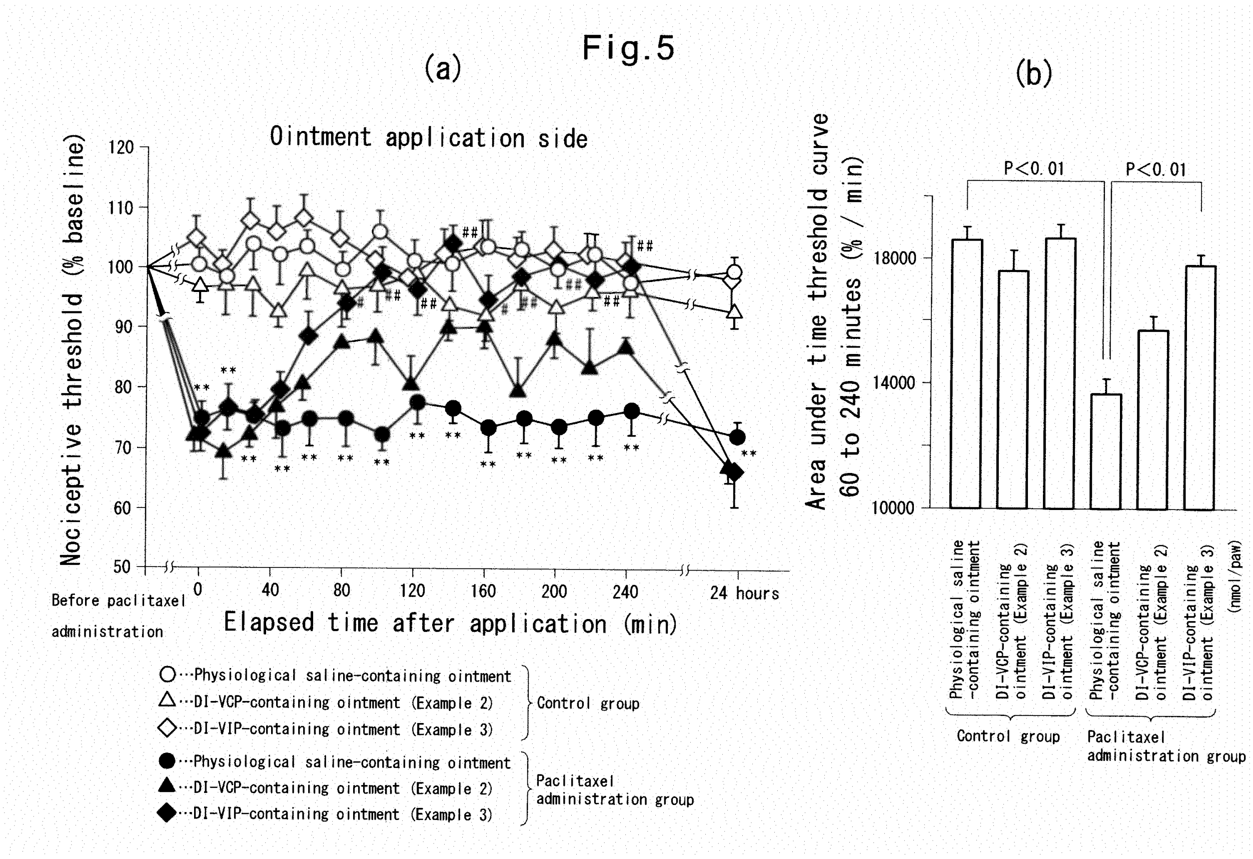
(a)
Ointment application side
Nociceptive threshold (% baseline)
120
110
100
90
80
70
60
50
Before paclitaxel administration
0
40
80
120
160
200
240
24 hours
Elapsed time after application (min)
○···Physiological saline-containing ointment
△···DI-VCP-containing ointment (Example 2)
◇···DI-VIP-containing ointment (Example 3)
Control group
●···Physiological saline-containing ointment
▲···DI-VCP-containing ointment (Example 2)
◆···DI-VIP-containing ointment (Example 3)
Paclitaxel administration group
(b)
Area under time threshold curve 60 to 240 minutes (% / min)
P<0.01
P<0.01
18000
14000
10000
Physiological saline -containing ointment
DI-VCP-containing ointment (Example 2)
DI-VIP-containing ointment (Example 3)
Physiological saline -containing ointment
DI-VCP-containing ointment (Example 2)
DI-VIP-containing ointment (Example 3)
(nmol/paw)
Control group
Paclitaxel administration group

Fig.6

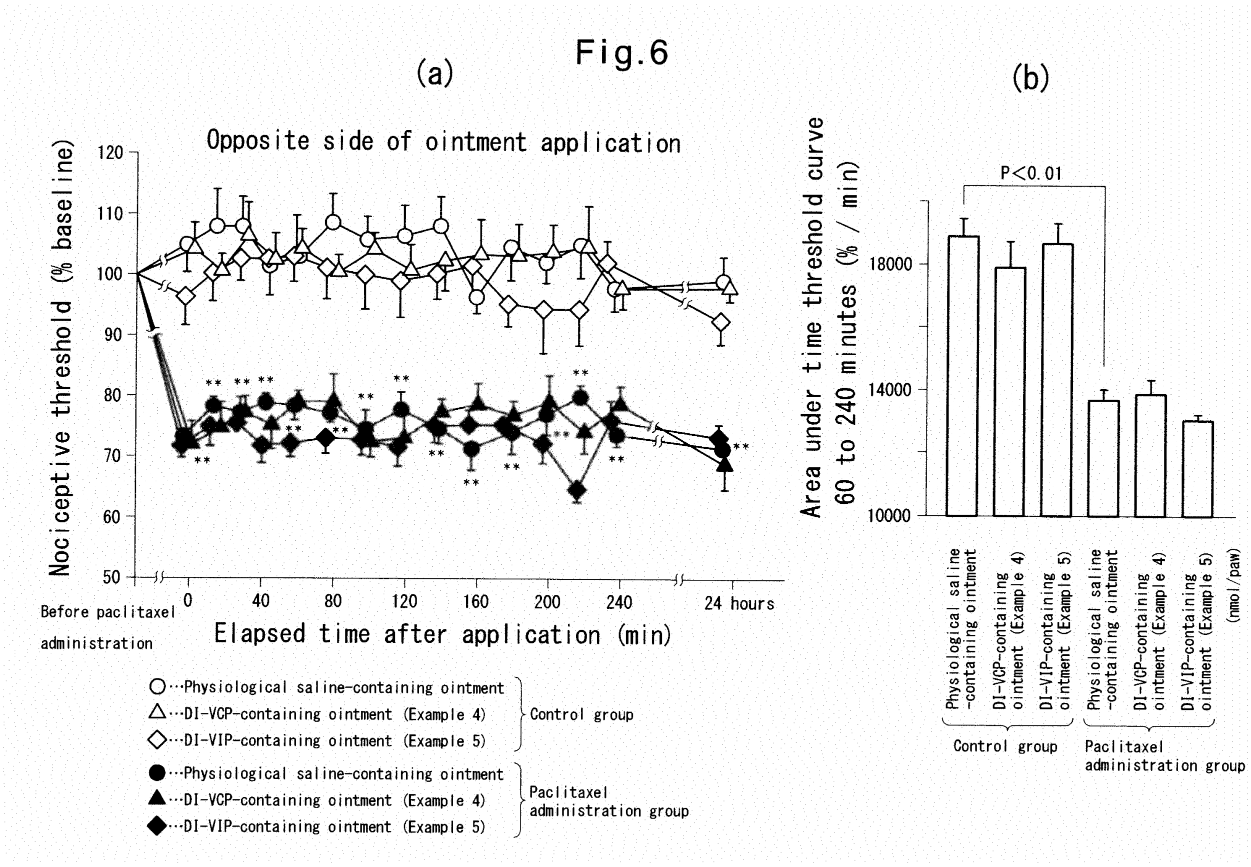
(a)
Opposite side of ointment application
Nociceptive threshold (% baseline)
120
110
100
90
80
70
60
50
Before paclitaxel administration
0
40
80
120
160
200
240
24 hours
Elapsed time after application (min)
**
○···Physiological saline-containing ointment
△···DI-VCP-containing ointment (Example 4)
◇···DI-VIP-containing ointment (Example 5)
Control group
●···Physiological saline-containing ointment
▲···DI-VCP-containing ointment (Example 4)
◆···DI-VIP-containing ointment (Example 5)
Paclitaxel administration group
(b)
Area under time threshold curve 60 to 240 minutes (% / min)
P<0.01
18000
14000
10000
Physiological saline -containing ointment
DI-VCP-containing ointment (Example 4)
DI-VIP-containing ointment (Example 5)
Physiological saline -containing ointment
DI-VCP-containing ointment (Example 4)
DI-VIP-containing ointment (Example 5)
(nmol/paw)
Control group
Paclitaxel administration group

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 52018191 B
- JP 3139288 A
- WO 2005092905 A

### Non-patent literature cited in the description


- Classification of chronic pain. IASP. IASP Press, 2002, 210
- **KAWABATA et al.** *Pain,* 2007, vol. 132, 74-81
- **MAEDA et al.** *Pain,* 2009, vol. 142, 127
- **TAKAHASHI et al.** *J Pharmacol Sci,* 2009, vol. 109 (1), 190
- **MATSUNAMI et al.** *Gut,* 2009, vol. 58, 751
- **NISHIMURA et al.** *Gut,* 2009, vol. 58, 762
- **NELSON et al.** *J Neurosci,* 2007, vol. 27, 12577-83
- **OKUBO et al.** *Journal of pharmaceutical sciences,* 2009, vol. 109 (1), 222P
- **NELSON et al.** *Journal of Neuroscience,* 2007, vol. 27, 12577-12583
- **POLOMANO et al.** *Pain,* 2001, vol. 94, 293-304